(19) Europäisches Patentamt

European Patent Office

Office européen des brevets

(11) EP 1 709 982 A1

(12) EUROPEAN PATENT APPLICATION
published in accordance with Art. 158(3) EPC

(43) Date of publication:
11.10.2006 Bulletin 2006/41

(51) Int Cl.:
A61M 1/14 (2006.01)        A61M 1/34 (2006.01)

(21) Application number: 05709269.4

(22) Date of filing: 24.01.2005

(86) International application number:
PCT/JP2005/000825

(87) International publication number:
WO 2005/070478 (04.08.2005 Gazette 2005/31)

(84) Designated Contracting States:
DE FR GB NL SE

(30) Priority: 27.01.2004 JP 2004017738
26.10.2004 JP 2004311027

(71) Applicant: Japan Science and Technology Agency
Kawaguchi-shi,
Saitama 332-0012 (JP)

(72) Inventors:
• UMEHARA, Yutaka
0368183 (JP)
• UMEHARA, Minoru
0380004 (JP)
• Sasaki, Mutsuo
0368227 (JP)

(74) Representative: Strehl Schübel-Hopf & Partner
Maximilianstrasse 54
80538 München (DE)

(54) PLASMA EXCHANGE WASTE LIQUID PURIFICATION CIRCULATION DIALYZER

(57) The object of the invention is to provide an apparatus used in a plasma exchange therapy, which is operable with only a minor modification to a conventional plasma exchange apparatus and wherein valuable plasma is not disposed but recycled as a dialysis solution.

The present invention is related to an apparatus used in a plasma exchange therapy wherein at least a portion of separated plasma is purified and circulated as a dialysis solution to dialyze patient's blood to remove harmful substances contained in the plasma; a plasma exchange apparatus incorporating said apparatus; and an artificial liver equipped with these apparatuses.

FIG. 1

**Description**

FIELD OF THE INVENTION

The invention relates to a plasma exchange waste liquid purification and circulation dialysis apparatus wherein at least a portion of plasma separated from a patient is purified and circulated as a dialysis solution to dialyze the patient's blood to remove harmful substances contained in the plasma.

BACKGROUND ART

A plasma exchange therapy (plasma separation and exchange therapy) wherein patient's plasma is replaced by fresh and frozen plasma obtained from a normal individual will be adopted when other kinds of therapy demonstrated no or insufficient effect for disorders caused by an abnormal antibody, an immuno-complex or an overproduced normal factor such as, for example, acute hepatic insufficiency, anti-glomerular basement membrane antibody diseases such as Goodpasture syndrome, amyosthenia, Guillain-Barre syndrome, necrotizing angitis, thrombopenic purpura thrombotic, and familial cholesteremia.
Representative examples of said plasma exchange therapy are listed as follows:

[Simple plasma exchange therapy]

It is impossible to infuse fresh plasma after the removal of all of the plasma components, which would mean "exchange" in a true sense, due to the necessity of maintenance of body function. Accordingly, hematocytes and plasma are separated by centrifugal force applied at a hemofilter or plasma separator, and the fresh and frozen plasma will be infused while the patient's plasma thus separated being disposed. However, since the patient' s plasma will always be separated and disposed only after having been mixed with the infused fresh plasma in body, it is presumed that the composition of the patient's plasma after the exchange of a certain amount of plasma will be almost the same as that of the plasma disposed. That is to say, this "plasma exchange therapy" is actually no more than dilution of the patient's plasma with a certain amount of the fresh plasma, and disposal of surplus of the plasma.

[Double-filtration plasma exchange therapy]

This method removes plasma proteins more selectively than in the simple plasma exchange therapy, and recycles a portion of the plasma and uses an albumin solution as a supplementing liquid. However, as it requires separation by means of molecular weight fraction, there is a possibility that necessary proteins may be removed as well.
The following "Plasma adsorption therapy" and "MARS" may be also mentioned as representative examples of the method for removing disease agent and toxic substances in plasma.

[Plasma adsorption therapy]

Almost all kinds of adsorbents will require perfusion in a plasma state for fear of blood clot, decrease of platelets and hemolysis. It is therefore the plasma will be in contact with an adsorbent after it is separated from hematocytes so that disease agent in the plasma may be selectively removed. However, this therapy has to be sometimes combined with the simple plasma exchange therapy depending on conditions such as in acute hepatic insufficiency. Accordingly, a therapy that can be more simply combined with other kinds of therapy is required for view of complexity of apparatuses and complication of procedures.

[Molecular Adsorbent Recycling System ("MARS" trade mark)]

This was developed for the purpose of selectively removing protein-binding toxic substances using the albumin solution as the dialysis solution in the treatment of acute hepatic insufficiency. Random sampling tests are currently conducted in facilities mainly in Germany and France. The removing efficiency greatly depends on a dialysis membrane, as the toxic substances binding to the albumin can permeate it while albumin itself cannot permeate the membrane. Since there is an objection to a concept of this therapy, it seems that it will take more time for the therapy to be recognized as a new technology.

Non-Patent document 1 :
Umehara, et al., LIVERAID TM-A Novel Hybrid Bioartificial Liver-Hepatology 2002, 36(4) suppl.:681A
Non-Patent document 2 :

Watanabe et al. , Clinical experience with a bioartificial liver in the treatment of severe liver failure- A pahse I clinical Trial- Annals of Surgery 1997: 225 (5): 484-494

Non-Patent document 3 :

Stange, et al., Molecular Adsorbent Recycling System (MARS) : Clinical results of a new membrane based blood purification system for bioartificial liver suppport, Artif Organs 1999:23 (4):319-330

## SUMMARY OF THE INVENTION

## PROBLEMS TO BE SOLVED BY THE INVENTION

All of the fresh and frozen plasma is supplied from blood donated by volunteers now in Japan. Accordingly, a method to utilize more effectively such valuable material obtained from human body in the plasma exchange therapy.

The present inventors have studied hard to solve the above problems, and finally found that plasma exchange waste liquid (separated plasma) may be utilized in a secondary therapy, more specifically used as a dialysis solution after being purified. The present invention was made on the basis of the above findings.

## MEANS FOR SOLVING THE PROBLEM

Thus, the present invention relates to a plasma exchange waste liquid purification and circulation dialysis apparatus wherein at least a portion of separated plasma is purified and circulated as a dialysis solution to dialyze patient's blood to remove harmful substances contained in the plasma. As already mentioned above, the representative examples of the plasma exchange therapy include the simple plasma exchange therapy and double-filtration plasma exchange therapy. The apparatus according to the present invention may be used widely in any plasma exchange therapy in addition to the above methods.

In one example, the apparatus comprises a purifier and dialyzer as their constituent elements. Dialysis of patient's blood with the separated plasma circulated through the dialyzer may be performed by any means or method known for those skilled in the art. The dialyzer (dialysis apparatus) may be equipped independently from an apparatus for plasma separation such as a plasma separator or hemofilter that are used in the plasma exchange, or more conveniently the apparatus for plasma separation may function also as the dialyzer.

Purification of the separated plasma may be preformed at the purifier (bioreactor) by any means or method known for those skilled in the art. Various harmful substances such as disease agents and toxic substances can be removed from the separated plasma by the purifier. For example, when the purifier is composed of an adsorption separator, various harmful substances binding to the plasma proteins will be adsorbed to any adsorbent equipped with the adsorption separator and removed from the plasma by the action of adsorbents such as various ion-exchange resins; various porous copolymers synthesized from divinyl-benzene, divinyl-toluene and styrene; activated carbon; and alumina.

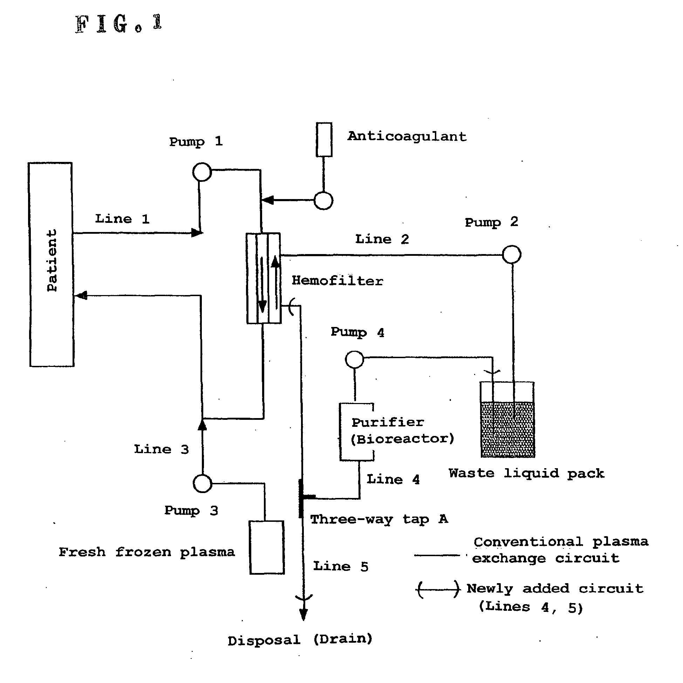

A preferable example of the present invention may be so constructed that the separated plasma functions as reservoir of the dialysis solution. The apparatus of the present invention may comprise other elements known for those skilled in the art, such as a pump, a line and a three-way tap. One example of the apparatus of the present invention is composed of a hemofilter, a purifier, a three-way tap, a pump (4), a line (4) and a line (5) shown in Fig.1.

The plasma exchange waste liquid purification and circulation dialysis apparatus according to the present invention may be easily added to any type of the plasma exchange apparatuses and used as a part thereof. It is therefore that the present invention relates also to a plasma exchange apparatus incorporating the plasma exchange waste liquid purification and circulation dialysis apparatus of the present invention.

An artificial liver using liver cells or tissue is now being developed in order to be used in the treatment of acute hepatic insufficiency or as an intermediary for liver transplantation. Many of them adopt a system wherein plasma is separated, oxygenated, and induced into a bioreactor, mixed with hematocytes and brought back into a patient's body. It has been reported that the plasma of the patient suffering from acute hepatic insufficiency comprises components that will impair the function of the liver cells or tissue. It seems therefore very effective that the concentration of the above harmful substances is decreased by means of the plasma exchange therapy before the treatment using the bioreactor with the liver cells or tissue.

The present invention is therefore related to an artificial liver including a hybrid type equipped with the plasma exchange waste liquid purification and circulation dialysis apparatus of the present invention. Such artificial liver may serve as a platform for various types of the bioreactor in the development of the artificial liver.

## ADVANTAGES OF THE INVENTION

It is possible to effectively remove the harmful substances contained in the plasma by recycling the plasma as the dialysis solution without being disposed with the use of the apparatus according to the present invention. Furthermore, as the apparatus of the present invention is operable with only a minor modification to a conventional plasma exchange apparatus, other therapy such as the plasma adsorption therapy can be quickly initiated after the simple plasma exchange therapy is finished. Priming volume of an apparatus will often make some problems in blood purification therapy. However, since the plasma exchange waste liquid may function as reservoir of the dialysis solution and a priming solution in the apparatus of the present invention, it can be used very safely even for patients who are instable in circulatory dynamics or have small body such as children.

BRIEF DESCRIPTION OF DRAWINGS

Fig.1 shows an example of the present apparatus.

Fig.2 shows a circuit of the example at the time of plasma exchange (PE).

Fig.3 shows a circuit of the example at the time of priming by plasma exchange waste liquid of the purifier.

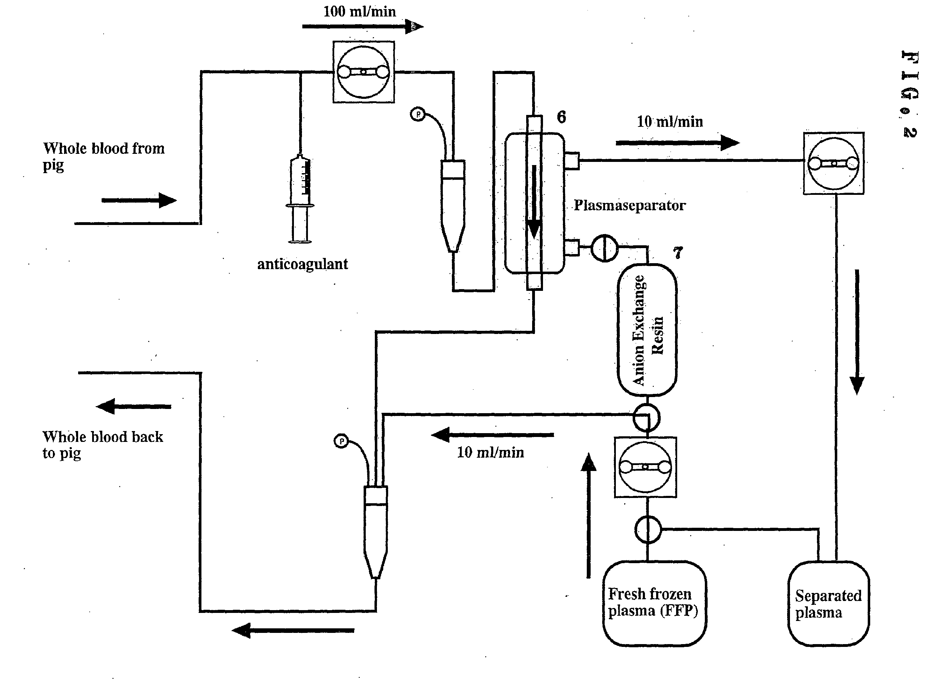

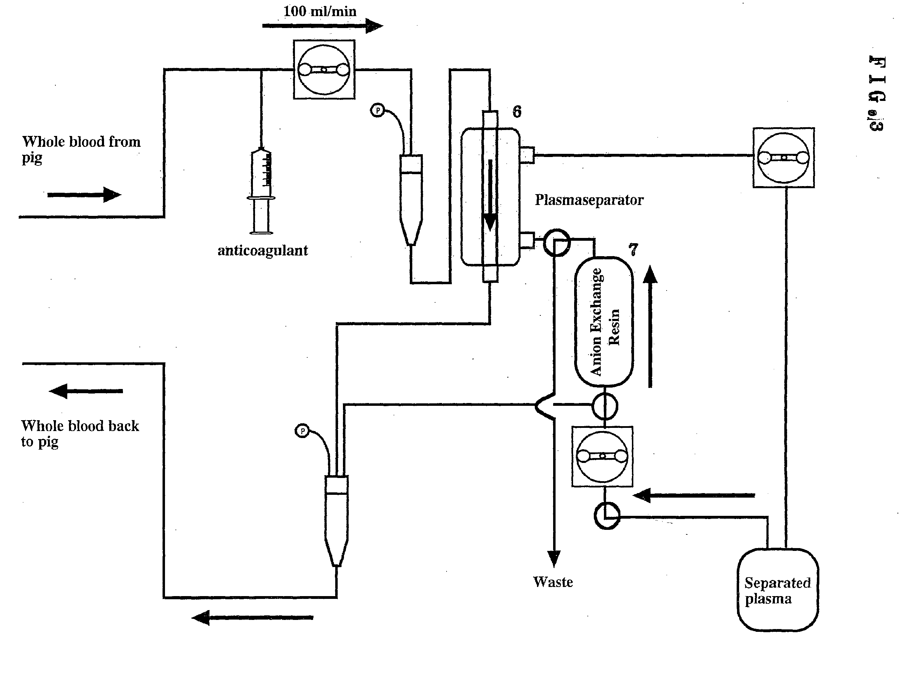

Fig.4 shows a circuit of the example at the time of plasma exchange waste liquid purification and circulation dialysis (Plasma Recycling Dialysis:PRD).

Fig.5 is a graph showing changes in the value of a total bile acid (TBA) in the plasma and the plasma exchange waste liquid at 0.5, 1 and 2 hours after the initiation of the plasma exchange (PE). The value in the plasma before PE is set as "100" and the other values are shown as % for said value.

Fig.6 is a graph showing changes in the value of a total bilirubin(T-Bil) in the plasma and the plasma exchange waste liquid at 0.5, 1 and 2 hours after the initiation of the plasma exchange (PE). The value in the plasma before PE is set as "100" and the other values are shown as % for said value.

Fig.7 is a graph showing changes in blood pressure (Systolic pressure) before or after the transition from PE to PRD.

Fig.8 is a graph showing changes in the number of white blood cell (WBC) through PE and PRD.

Fig. 9 is a graph showing changes in the number of red blood cell (RBC) through PE and PRD.

Fig.10 is a graph showing changes in the value of hemoglobin (Hb) through PE and PRD.

Fig.11 is a graph showing changes in the value of hematocrit (Ht) through PE and PRD.

Fig.12 is a graph showing changes in the number of platelet through PE and PRD.

Fig.13 is a graph showing changes in the value of TBA through PE and PRD.

Fig.14 is a graph showing changes in the value of T-Bil through PE and PRD.

EXPLANATION OF SYMBOLS

1:  Pump, Line;
2:  Pump, Line;
3:  Pump, Line;
4:  Pump, Line;
5:  Line;
6:  Plasma separator;
7:  Purifier (anion-exchange resin)

Best Mode for Carrying out the Invention

One example of the present invention will be explained with reference to Fig.1. The numbers in the specification correspond to those in the figure. Those skilled in the art may easily conceive various aspects having the technical features of the present invention based on the disclosure of the present specification and technical common sense in the art, and it is clear and obvious that such various aspects shall therefore fall within the scope of the present invention.

Patient's blood is first introduced (100 ml/min) into the hemofilter (that will serve also as the dialyzer) through a line (1) by means of a pump (1). The plasma exchange waste liquid is separated and passed (5-10 ml/min) through a pump (2) to a waste liquid pack. Simultaneously, fresh frozen plasma (FFP) is perfused into the patient at the same rate through a line (3) by means of a pump (3). A pump (4) is stopped, and lines (4) and (5) are closed with a three-way tap (A) during the above procedures. After a predetermined amount of the plasma has been exchanged, the pumps (2) and (3) are stopped, and the lines (4) and (5) are opened, and then a predetermined amount of the plasma exchange waste liquid in the waste liquid pack is disposed through the line (5) by means of the pump (4). For example, one (L) of the plasma exchange waste liquid out of 2 (L) in total is disposed and the other one(L) is left in the waste liquid pack (Priming by the plasma exchange waste liquid of the line (4) and the purifier). The line (5) is then closed by the three-way tap (A). The pumps (2) and (4) are then rotated at the same rate (for example, 400 ml/min) to purify the plasma exchange waste liquid and dialyze the plasma.

Any element known for those skilled in the art may be used as the constituent elements used in the above apparatus, such as the apparatus for plasma separation (the plasma separator or hemofilter), the pump, and the line. Furthermore, those skilled in the art may use or operate the apparatus shown in Fig.1 without any difficulty.

EXAMPLE

Examples of the plasma exchange and the plasma exchange waste liquid purification and circulation dialysis (Plasma Recycling Dialysis:PRD) with the apparatus according to the present invention shall be shown by an animal experiment using a hyper-bilirubin plasma pig model. The following examples will not limit in any sense the scope of the present invention.

Method

[Experimental animal]

A fatted female pig (25-30 kg body weight) was bred in a metal breeding gauge for the experiment. The animal was bred under sunshine of from eight to twenty o'clock at a constant temperature and humidity ($22\pm2$ °C, $55\pm10\%$) and free to take tap water. All of the experiment were conducted in accordance with "Guideline for animal experiments at Hirosaki University." All of the operations and extracorporeal circulation were done under general anesthesia as follows. The animal was fasted except free uptake of water from the night before the operation. Ketamine (15 mg/kg) and Atropine (0.01 mg/kg) were intramuscularly injected. After the pig had fallen asleep, a blood vessel was secured from an auricular vein. After thiopentanol sodium (100mg) was administered, intratracheal intubation was done. Anesthesia was kept by administrating Midazolam (0.3 mg/kg/h), Pentazocine (0.3 mg/kg/h) and pancuronium bromide (0.4 mg/kg/h) under artificial respiration using a ventilator

Hyper-bilirubin plasma pig model

The pig model was prepared under general anesthesia by subjecting to an abdominal operation at median and upper abdomen, extracting a gall bladder, and ligating and cutting of choledoch duct (common bile duct). The course after the operation was observed while the animal was bred in the gauge. Seven days later, under general anesthesia, a double lumen catheter for extracorporeal circulation (GamCath, Gambro, Stockholm) was inserted into a right cervical vein and a catheter was inserted into a right femoral artery for measurement of blood pressure and collection of blood.

Plasma exchange (PE)

The plasma separator (ACH-07S, Asahi Medical, Tokyo) was used for the plasma exchange and the plasma exchange waste liquid purification and circulation dialysis. Anticoagulant (Heparin sodium) was intravenously administered at a dose of 6, 000 units before the extracorporeal circulation, and at a rate of 2, 000 units per hour after the initiation of extracorporeal circulation. A flow rate of blood was set to be 100 ml/min. The plasma exchange was carried by means of a membrane-type plasma separator (OP-05W, Asahi Medical, Tokyo) using a supplementing solution of swine fresh frozen plasma at an amount (L) calculated by the following formula out for 2 hours (10 - 14 ml/min)(Fig.2):

$$\texttt{body weight (kg) x 1/13 x (1-Ht/100)}$$

Priming of the purifier with plasma exchange waste liquid

After the completion of the plasma exchange, a filter pump was stopped and a flow path was changed by means of the three-way tap. Then, the purifier was primed with the plasma exchange waste liquid (50 ml/min, 300 ml) by means of a supplementing solution pump (Fig.3).

The plasma exchange waste liquid purification and circulation dialysis (Plasma Recycling Dialysis:PRD)

After the completion of the priming, the flow path was changed by means of the three-way tap. PRD was carried out for 6 hours using the plasma separator as the dialyzer, which had been used for the plasma separation in PE (Fig.

4). Anion-exchange resin (Plasorba BRS-350, Asahi Medical, Tokyo) was used as the purifier. A flow rate of blood was set to be 100 ml/min and the plasma exchange waste liquid was circulated at a rate of 50 ml/min through an outer space of hollow fiber countercurrently against a blood flow. After the completion, the extracorporeal circulation was stopped and the blood was returned.

Measurement items:

Artery pressure was continuously measured with the catheter inserted into the femoral artery. Blood was collected before the initiation of PE; at 0. 5, 1, and 2 hours after the initiation of PE; and at 2, 4, 6 (end of PRD) and at 8 hours after the initiation of PRD, and subjected to blood examination with respect to total protein, albumin, Na, Cl, K, urea nitrogen, total bile acid, total bilirubin, Creatinin, LDH, AST, and ALT (n=4). The plasma exchange waste liquid was also colleted at 0.5, 1, and 2 hours after the initiation of PE, followed by determination of a total bile acid and total bilirubin concentration (n=4).

Results:

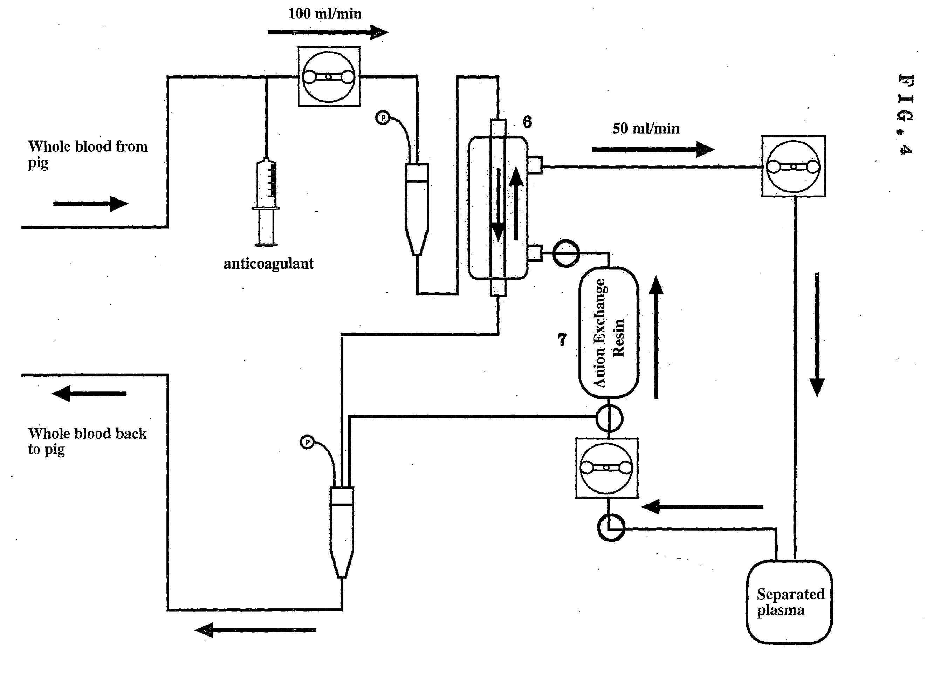

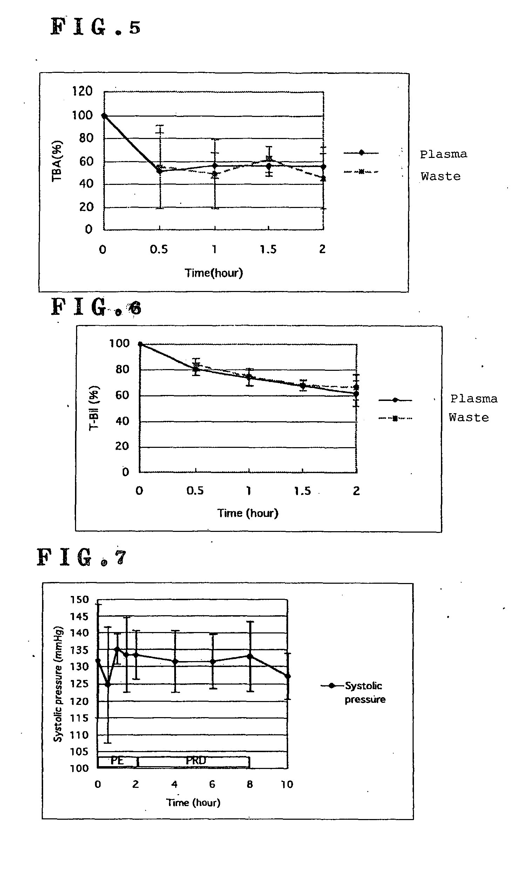

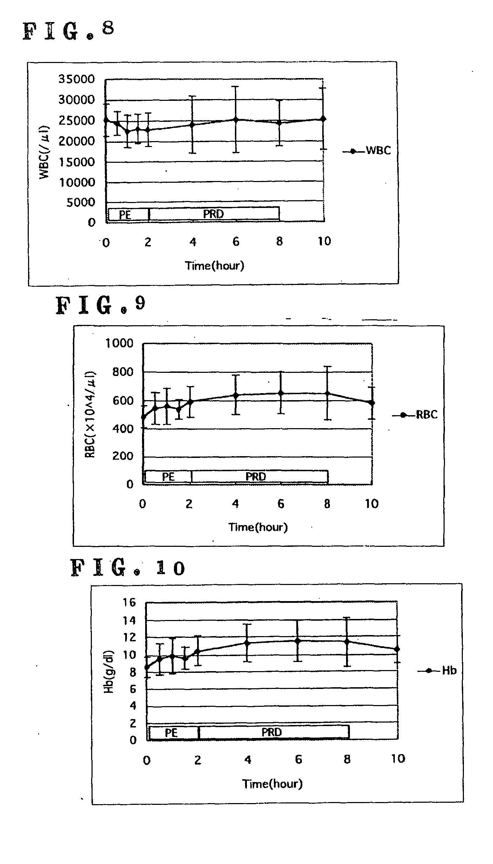

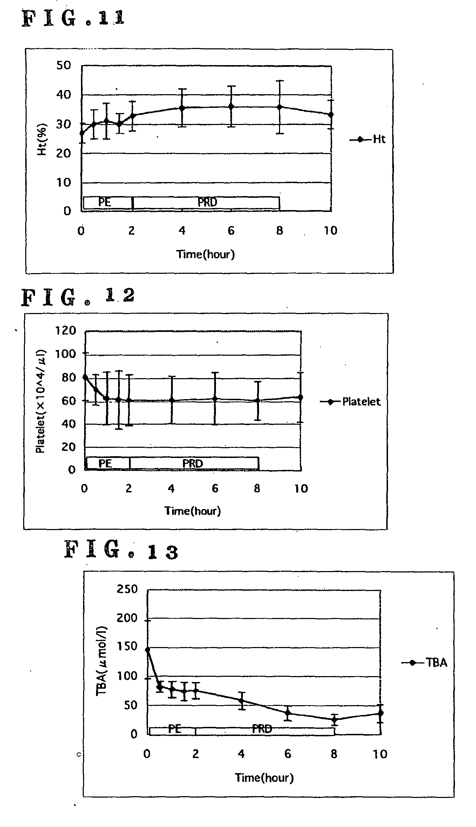

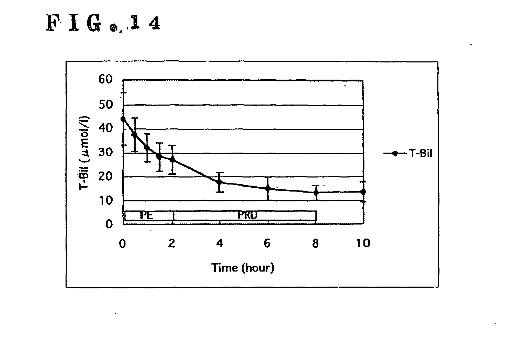

It was confirmed that the values of the total bile acid and the total bilirubin were almost the same in the plasma and the plasma exchange waste liquid colleted at 0.5, 1, and 2 hours after the initiation of PE (Fig. 5 & 6). Circulatory dynamics was stable until the end of extracorporeal circulation, and no change in blood pressure (systolic pressure) was observed before and after the transition from PE to PRD (Fig.7). No hemolysis was observed through PE and PRD. The blood examination showed a little increase after the end of the plasma exchange with respect to the numbers of white blood cell (WBC) and red blood cell (RBC), and the values of hemoglobin and hematocrit (Fig.8 - Fig.11). The number of platelet was decreased in PE, but became stable after the transition to PRD (Fig. 12). Biochemical examination showed that the values of the total bile acid and the total bilirubin were continuously decreased even after the transition from PE to PRD (Fig.13 & Fig.14). No significant change was observed with respect to the other values through PE and PRD (TABLE 1).
[TABLE 1]

|  | Before PE | End of PE | End of PRD |
|---|---|---|---|
| Total Protein (g/dl) | $5.7 \pm 0.5$ | $5.0 \pm 0.1$ | $5.0 \pm 0.3$ |
| Albumin (mg/dl) | $2.7 \pm 0.2$ | $2.4 \pm 0.1$ | $2.4 \pm 0.2$ |
| AST (IU/L) | $31 \pm 13$ | $36 \pm 12$ | $36 \pm 8$ |
| ALT (IU/L) | $18 \pm 4$ | $23 \pm 3$ | $23 \pm 2$ |
| LDH (IU/L) | $371 \pm 51$ | $430 \pm 34$ | $435 \pm 100$ |
| BUN (mg/dl) | $6.8 \pm 1.9$ | $7.4 \pm 1.8$ | $8.8 \pm 3.0$ |
| Creatinin (mg/dl) | $0.8 \pm 0.1$ | $0.7 \pm 0.1$ | $0.6 \pm 0.1$ |
| Na (mEq/L) | $138 \pm 1$ | $138 \pm 2$ | $135 \pm 2$ |
| K (mEq/L) | $4.2 \pm 0.3$ | $3.6 \pm 0.3$ | $3.9 \pm 0.5$ |

[Discussion]

The above examples demonstrated the safety of the combination of PE and PRD, and the possibility of transition or shift from PE to PRD in a short time. In the apparatus according to the present invention, the plasma exchange waste liquid functions as the reservoir and the function of the purifier is supplemented via plasma dialysis. It was also confirmed that such the apparatus of the present invention could effect a sufficient removing efficiency of the harmful substances in the plasma as shown by the changes of the values of the harmful substances in the plasma such as those of the total bilirubin and the total bile acid. Although the anion-exchange resin was used as the purifier in the above examples, it is conceived that any kind of the combination of various adsorbents and hemodialysis may be used as well, and that the purifier may be easily scaled up or exchanged on the way of the therapy. It is also conceived that the apparatus according to the present invention enables a low-invasive and rapid transition from PE to the secondary therapy and is therefore useful as an artificial liver support.

[Industrial Applicability]

The plasma exchange therapy still has an important place in the therapy of acute liver insufficiency in Japan. The same therapy has a long history and is very safe so that it is considered a therapy that can be performed with little hesitation. This therapy is actually performed in many medical facilities. As the apparatus of the present invention may be very easily composed with a minor modification of the addition of the purifier to the plasma exchange therapy, it is a very useful system that may be easily introduced by doctors skilled in the plasma exchange therapy.

**Claims**

1. A plasma exchange waste liquid purification and circulation dialysis apparatus wherein at least a portion of separated plasma is purified and circulated as a dialysis solution to dialyze patient's blood to remove harmful substances contained in the plasma.

2. The apparatus of Claim 1, which is used in a simple plasma exchange therapy.

3. The apparatus of Claim 1, which is used in a double-filtration plasma exchange therapy.

4. The apparatus of Claim 1, comprising a purifier and a dialyzer as a constituent element of the apparatus.

5. The apparatus of Claim 4 wherein an apparatus for plasma separation that are used in the plasma exchange functions also as the dialyzer.

6. The apparatus of Claim 4 or 5 wherein the purifier is composed of an adsorption separator.

7. The apparatus of one of Claims 1 to 6 wherein the plasma separated in the plasma exchange therapy functions as reservoir of the dialysis solution.

8. The apparatus of one of Claims 1 to 7, being composed of a hemofilter, a purifier, a three-way tap (A), a pump (4), a line (4) and a line (5) shown in Fig.1.

9. A plasma exchange apparatus incorporating the plasma exchange waste liquid purification and circulation dialysis apparatus according to any one of the preceding claims.

10. The apparatus having the composition shown in Fig. 1.

11. An artificial liver equipped with the apparatus according to any one of the preceding claims.

**F I G . 1**

**Anticoagulant**

Pump 1

Line 1

Patient

Pump 2

Line 2

Hemofilter

Pump 4

Line 3

Purifier
(Bioreactor)

Waste liquid pack

Pump 3

Line 4

Fresh frozen plasma

Three-way tap A

Line 5

Conventional plasma
exchange circuit

Newly added circuit
(Lines 4, 5)

Disposal (Drain)

# F I G. 2

FIG.3

F I G . 4

# F I G . 5

# F I G . 6

# F I G . 7

## FIG. 8

## FIG. 9

## FIG. 10

FIG.11

FIG.12

FIG.13

# FIG. 14

## INTERNATIONAL SEARCH REPORT

| | International application No. |
|---|---|
| | PCT/JP2005/000825 |

| A. CLASSIFICATION OF SUBJECT MATTER |
|---|
| Int.Cl$^7$  A61M1/14, 1/34 |

According to International Patent Classification (IPC) or to both national classification and IPC

| B.  FIELDS SEARCHED |
|---|
| Minimum documentation searched (classification system followed by classification symbols) |
| Int.Cl$^7$  A61M1/02-1/36 |

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

| C.  DOCUMENTS CONSIDERED TO BE RELEVANT | | |
|---|---|---|
| Category* | Citation of document, with indication, where appropriate, of the relevant passages | Relevant to claim No. |
| X | JP 9-507414 A  (HemoCleanse, Inc.), 29 July, 1997 (29.07.97), Full text; all drawings & WO 95/18671 A        & US 5536412 A | 1-7,9,11 |
| A | JP 7-506765 A  (STANGE, Jan), 27 July, 1995 (27.07.95), Full text; all drawings & WO 94/21363 A | 1-7,9,11 |

| ☐ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 09 February, 2005 (09.02.05) | 01 March, 2005 (01.03.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

## INTERNATIONAL SEARCH REPORT

| International application No. |
| --- |
| PCT/JP2005/000825 |

**Box No. II        Observations where certain claims were found unsearchable (Continuation of item 2 of first sheet)**

This international search report has not been established in respect of certain claims under Article 17(2)(a) for the following reasons:

1. ☐  Claims Nos.:
   because they relate to subject matter not required to be searched by this Authority, namely:

2. ☒  Claims Nos.:  8, 10
   because they relate to parts of the international application that do not comply with the prescribed requirements to such an
   extent that no meaningful international search can be carried out, specifically:
   The subject matter of the inventions of claims 8 and 10 cannot be identified
   because although claims 8 and 10 contain the language "shown in Fig. 1", it
   is probable that drawings are interpreted in multisense.

3. ☐  Claims Nos.:
   because they are dependent claims and are not drafted in accordance with the second and third sentences of Rule 6.4(a).

**Box No. III        Observations where unity of invention is lacking (Continuation of item 3 of first sheet)**

This International Searching Authority found multiple inventions in this international application, as follows:

1. ☐  As all required additional search fees were timely paid by the applicant, this international search report covers all searchable
   claims.

2. ☐  As all searchable claims could be searched without effort justifying an additional fee, this Authority did not invite payment of
   any additional fee.

3. ☐  As only some of the required additional search fees were timely paid by the applicant, this international search report covers
   only those claims for which fees were paid, specifically claims Nos.:

4. ☐  No required additional search fees were timely paid by the applicant.   Consequently, this international search report is
   restricted to the invention first mentioned in the claims; it is covered by claims Nos.:

**Remark on Protest**        ☐  The additional search fees were accompanied by the applicant's protest.

☐  No protest accompanied the payment of additional search fees.

Form PCT/ISA/210 (continuation of first sheet (2)) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **UMEHARA et al.** LIVERAID TM-A Novel Hybrid Bi-oartificial Liver. *Hepatology,* 2002, vol. 36 (4), 681A
- **WATANABE et al.** Clinical experience with a bioartificial liver in the treatment of severe liver failure- A pahse I clinical Trial. *Annals of Surgery,* 1997, vol. 225 (5), 484-494

- **STANGE et al.** Molecular Adsorbent Recycling System (MARS) : Clinical results of a new membrane based blood purification system for bioartificial liver suppport. *Artif Organs,* 1999, vol. 23 (4), 319-330